(19) Europäisches Patentamt
European Patent Office
Office européen des brevets

(11) Veröffentlichungsnummer: **0 109 476**
**A1**

# (12) EUROPÄISCHE PATENTANMELDUNG

(21) Anmeldenummer: **83102103.5**

(22) Anmeldetag: **04.03.83**

(51) Int. Cl.³: **C 07 D 249/08**
**A 01 N 43/64**

(30) Priorität: **16.03.82 DE 3209431**

(43) Veröffentlichungstag der Anmeldung:
**30.05.84 Patentblatt 84/22**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Krämer, Wolfgang, Dr.**
**Am Eckbusch 39/45**
**D-5600 Wuppertal(DE)**

(72) Erfinder: **Büchel, Karl Heinz, Prof. Dr.**
**Dabringhausener Strasse 42**
**D-5093 Burscheid(DE)**

(72) Erfinder: **Brandes, Wilhelm, Dr.**
**Eichendorffstrasse 3**
**D-5653 Leichlingen(DE)**

(72) Erfinder: **Frohberger, Paul-Ernst, Dr.**
**Willi-Baumeister Strasse 5**
**D-5090 Leverkusen(DE)**

(54) **Phenoxypropyltriazolyl-ketone und -carbinole, Verfahren zu ihrer Herstellung und ihre Verwendung als Fungizide, sowie Zwischenprodukte für ihre Herstellung.**

(57) Die Erfindung betrifft neue Phenoxypropyltriazolylketone und -carbinole, ein Verfahren zu ihrer Herstellung und ihre Verwendung als fungizide.

Die Verbindungen der Formel

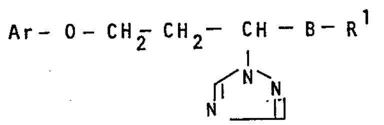

(I)

in welcher Ar, B und $R^1$ die in der Beschreibung angegebene Bedeutung besitzen, werden erhalten, wenn man z.B. Triazolyl-ketone mit Phenoxyethyl-Derivaten in Gegenwart einer Base und eines organischen Verdünnungsmittels umsetzt und gegebenenfalls die zunächst erhaltenen Keto-Derivate in üblicher Weise reduziert.

Die Wirkstoffe sind für den Gebrauch als Pflanzenschutzmittel geeignet und können mit besonders gutem Erfolg zur Bekämpfung von Venturia-Arten und zur Bekämpfung von Puccinia-Arten eingesetzt werden.

EP 0 109 476 A1

Croydon Printing Company Ltd.

BEZEICHNUNG GEÄNDERT
siehe Titelseite

BAYER AKTIENGESELLSCHAFT
Zentralbereich
Patente, Marken und Lizenzen

5090 Leverkusen, Bayerwerk

12. 03. 82
Slr/AB
Ia

## Phenoxypropyltriazolyl-ketone und -carbinole, Verfahren zu ihrer Herstellung und ihre Verwendung als Fungizide

Die vorliegende Erfindung betrifft neue Phenoxypropyltriazolyl-ketone und -carbinole, ein Verfahren zu ihrer Herstellung sowie ihre Verwendung als Fungizide.

Es ist bereits bekannt geworden, daß fluorierte 1-Triazolyl-butan-Derivate, wie z.B. 1-(4-Chlorphenoxy)-1-(1,2,4-triazol-1-yl)-3,3-dimethyl-butan-2-on bzw. -ol, 1-(4-Chlorphenoxy)-1-(1,2,4-triazol-1-yl)-3,3-bisfluormethyl-butan-1-on oder 1-(2,4-Dichlorphenoxy)-1-(1,2,4-triazol-1-yl)-3,3-dimethyl-butan-2-ol, im allgemeinen gute fungizide Eigenschaften aufweisen (vergleiche DE-OS 29 18 894 (LeA 19 618]). Die Wirkung ist jedoch, insbesondere bei niedrigen Aufwandmengen und -konzentrationen , in einigen Anwendungsbereichen nicht immer voll befriedigend.

Es wurden neue Phenoxypropyltriazolyl-ketone und -carbinole der allgemeinen Formel

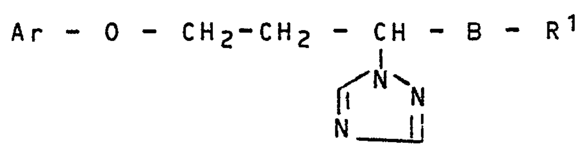

(I)

in welcher

Ar für gegebenenfalls substituiertes Phenyl steht,

B für die Ketogruppe oder eine CH(OH)-Gruppierung steht und

$R^1$ für substituiertes tert.-Butyl, gegebenenfalls substituiertes Phenyl oder gegebenenfalls substituiertes Cycloalkyl steht,

sowie deren Säureadditions-Salze und Metallsalz-Komplexe gefunden.

Diejenigen Verbindungen der Formel (I), in denen B für eine CH(OH)-Gruppierung steht, besitzen zwei asymmetrische Kohlenstoffatome; sie können deshalb in den beiden geometrischen Isomeren (erythro- und threo-Form) vorliegen, die in unterschiedlichen Mengenverhältnissen anfallen können. In beiden Fällen liegen sie als optische Isomeren vor.

Weiterhin wurde gefunden, daß man die neuen Phenoxypropyltriazolyl-ketone und -carbinole der Formel (I) erhält, wenn man Triazolyl-ketone der Formel

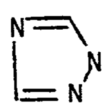 - $CH_2$ - CO - $R^1$ (II)

in welcher

$R^1$ die oben angegebene Bedeutung hat,

mit Phenoxyethyl-Derivaten der Formel

$$Ar - O - CH_2 - CH_2 - Z \qquad (III)$$

in welcher

Ar die oben angegebene Bedeutung hat und

Z für eine elektronenanziehende Abgangsgruppierung steht,

in Gegenwart einer Base und in Gegenwart eines organischen Verdünnungsmittels, oder in einem wässrig-organischen Zweiphasensystem in Gegenwart eines Phasentransferkatalysators, umsetzt und gegebenenfalls noch die so erhaltenen Keto-Derivate der Formel (I) nach bekannten Methoden in üblicher Weise reduziert.

An die so erhaltenen Verbindungen der Formel (I) kann gegebenenfalls anschließend eine Säure oder ein Metallsalz addiert werden.

Die neuen Phenoxypropyltriazolyl-ketone und -carbinole der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten fluorierten 1-Triazolyl-butan-Derivate, wie z.B. 1-(4-Chlorphenoxy)-1-(1,2,4-triazol-1-yl)-3,3-dimethyl-butan-2-on bzw. -ol, 1-(4-Chlorphenoxy)-1-(1,2,4-triazol-1-yl)-3,3-bis fluormethyl-butan-2-on oder 1-(2,4-Dichlorphenoxy)-1-(1,2,4-triazol-1-yl)-3,3-dimethyl-butan-2-ol, welche chemisch und wirkungsmäßig naheliegende Verbindungen sind. Die erfindungsgemäßen Stoffe stellen somit eine Bereicherung der Technik dar.

Die erfindungsgemäßen Phenoxypropyltriazolyl-ketone und -carbinole sind durch die Formel (I) allgemein definiert. In dieser Formel stehen vorzugsweise

Ar für gegebenenfalls einfach bis dreifach, gleich oder verschieden substituiertes Phenyl, wobei als Substituenten vorzugsweise genannt seien: Halogen, Alkyl mit 1 bis 4 Kohlenstoffatomen, Halogenalkyl mit 1 bis 2 Kohlenstoff- und 1 bis 5 gleichen oder verschiedenen Halogenatomen, wie Fluor- und Chloratomen, Alkoxy mit 1 bis 2 Kohlenstoffatomen, Nitro oder gegebenenfalls einfach bis zweifach durch Halogen substituiertes Phenyl;

B für die Ketogruppe oder eine CH(OH)-Gruppierung steht;

$R^1$ für gegebenenfalls einfach bis zweifach, gleich oder verschieden substituiertes Phenyl steht, wobei als Substituenten vorzugsweise die bei Ar bereits genannten Phenylsubstituenten infrage kommen, für jeweils gegebenenfalls einfach bis zweifach, gleich oder verschieden durch Methyl, Ethyl oder Isopropyl substituiertes Cyclopropyl, Cyclopentyl oder Cyclohexyl, sowie für die Gruppierungen

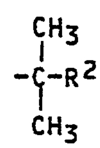 und 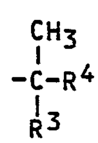 steht, wobei

$R^2$ für Alykl mit 2 bis 6 Kohlenstoffatomen, Halogenmethyl oder Alkenyl mit 2 bis 4 Kohlenstoffatomen steht und

$R^3$ und $R^4$ für Halogenmethyl stehen.

Besonders bevorzugt sind diejenigen Verbindungen der Formel (I), in denen

Ar für gegebenenfalls einfach bis zweifach, gleich oder verschieden substituiertes Phenyl steht, wobei als Substituenten vorzugsweise genannt seien: Fluor, Chlor, Brom, Methyl, Ethyl, Trifluormethyl, Pentafluorethyl, Methoxy, Ethoxy, Nitro, Phenyl und Chlorphenyl;

B für die Ketogruppe oder eine CH(OH)-Gruppierung steht;

$R^1$ für gegebenenfalls einfach bis zweifach, gleich oder verschieden substituiertes Phenyl steht, wobei als Substituenten vorzugsweise die bei Ar bereits genannten Phenylsubstitueneten infrage kommen, für jeweils gegebenenfalls einfach bis zweifach, gleich oder verschieden durch Methyl, Ethyl oder Isopropyl substituiertes Cyclopropyl, Cyclopentyl oder Cyclohexyl, sowie für die Gruppierungen

wobei $-\overset{CH_3}{\underset{CH_3}{\overset{|}{\underset{|}{C}}}}-R^2$ und $-\overset{CH_3}{\underset{R^3}{\overset{|}{\underset{|}{C}}}}-R^4$ steht,

$R^2$ für Alykl mit 2 bis 5 Kohlenstoffatomen, Fluormethyl, Chlormethyl oder Vinyl steht und

$R^3$ und $R^4$ für Fluormethyl oder Chlormethyl stehen.

Bevorzugte erfindungsgemäße Verbindungen sind auch Additionsprodukte aus Säuren und denjenigen Phenoxypropyltriazolyl-ketonen und -carbinolen der Formel (I), in denen die Substituenten Ar, B und $R^1$ die Bedeutungen haben, die bereits vorzugsweise für diese Reste genannt wurden.

Zu den Säuren die addiert werden könenn, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Salizylsäure, Sorbinsäure und Milchsäure, sowie Sulfonsäure, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Phenoxypropyltriazolyl-ketonen und -carbinolen der Formel (I), in denen die Substituenten Ar, B und $R^1$ die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuern ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der allgemeinen Formel (I) genannt:

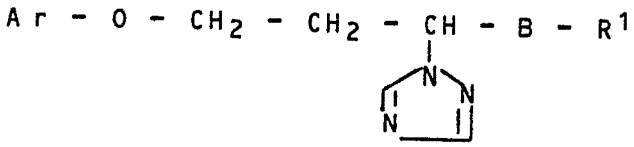 (I)

| Ar | B | $R^1$ |
| --- | --- | --- |
| 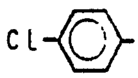 | CO | $-C(CH_3)_2CH_2Cl$ |
| 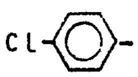 | CH(OH) | $-C(CH_3)_2CH_2Cl$ |
| 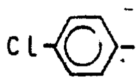 | CO | $-C(CH_2F)_2CH_3$ |
| 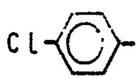 | CH(OH) | $-C(CH_2F)_2CH_3$ |
| 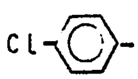 | CO | $-C(CH_2Cl)_2CH_3$ |
| 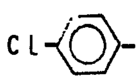 | CH(OH) | $-C(CH_2Cl)_2CH_3$ |
| 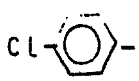 | CO | 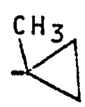 |
| 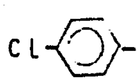 | CH(OH) | 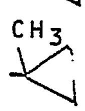 |
| 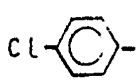 | CO | 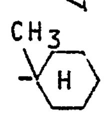 |
| 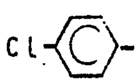 | CH(OH) | 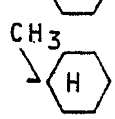 |
| 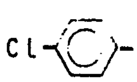 | CO | $-C(CH_3)_2CH=CH_2$ |
| 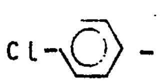 | CH(OH) | $-C(CH_3)_2CH=CH_2$ |

| Ar | B | $R^1$ |
|---|---|---|
| 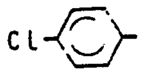 | CO | 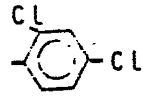 |
| 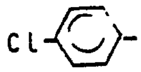 | CH(OH) | 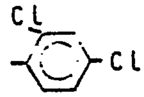 |
| 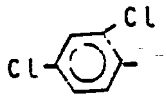 | CO | $-C(CH_3)_2CH=CH_2$ |
| 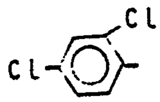 | CH(OH) | $-C(CH_3)_2CH=CH_2$ |
| 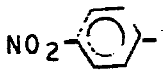 | CO | $-C(CH_3)_2CH(CH_3)_2$ |
| 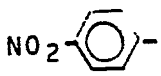 | CH(OH) | $-C(CH_3)_2CH(CH_3)_2$ |
| 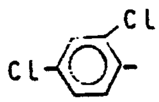 | CO | $-C(CH_3)_2CH(CH_3)_2$ |
| 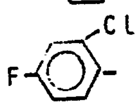 | CO | $-C(CH_3)_2CH_2F$ |
| 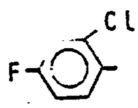 | CH(OH) | $-C(CH_3)_2CH_2F$ |
| 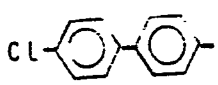 | CO | $-C(CH_3)_2CH_2F$ |
| 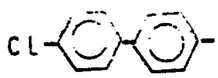 | CH(OH) | $-C(CH_3)_2CH_2F$ |
| 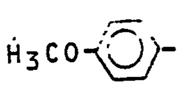 | CO | 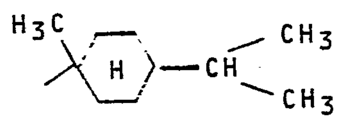 |
| 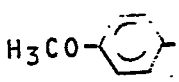 | CH(OH) | 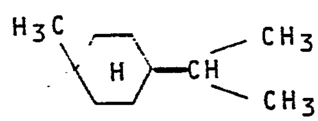 |
| 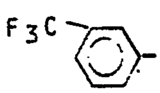 | CO | $-C(CH_3)_2-C_2H_5$ |
| 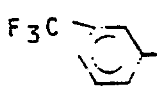 | CH(OH) | $-C(CH_3)_2-C_2H_5$ |

Verwendet man beispielsweise 3,3-Dimethyl-4-fluor-1-(1,2,4-triazol-1-yl)-2-butanon und 4-Chlorphenoxy-ethylbromid als Ausgangsstoffe, so kann der Reaktionsablauf des erfindungsgemäßen Verfahrens durch das folgende Formelschema wiedergegeben werden:

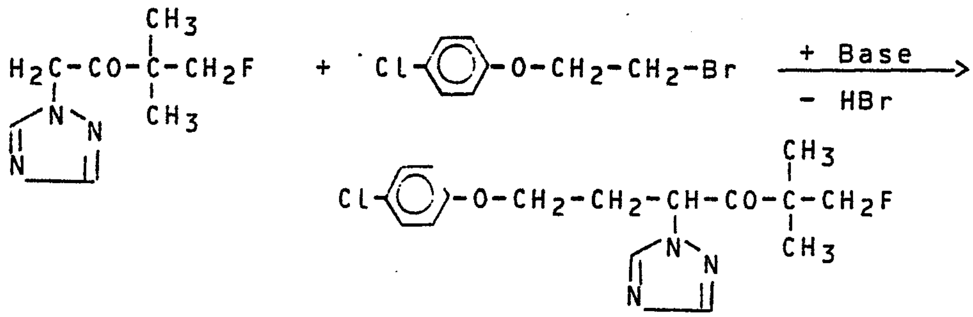


Verwendet man beispielsweise 6- (4-Chlorphenoxy)-2,2-dimethyl-1-fluor-4-(1,2,4-triazol-1-yl)-3-hexanon und Natriumborhydrid als Ausgangsstoffe, so kann der Reaktionsablauf der erfindungsgemäßen Reduktion durch das folgende Formelschema wiedergegeben werden:

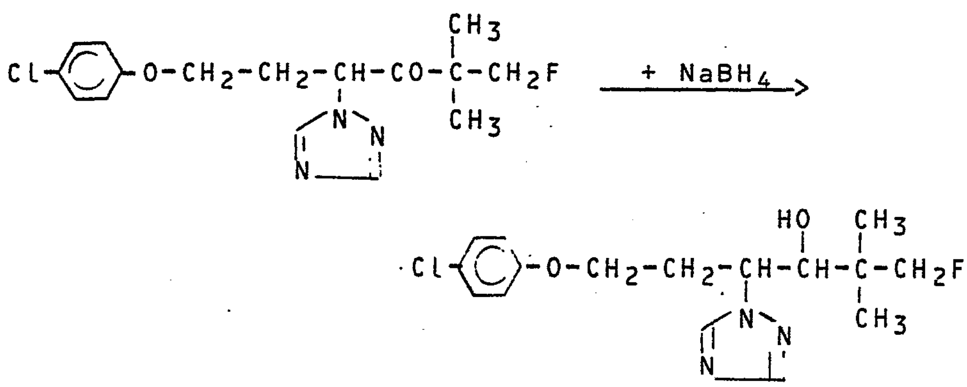

Die bei der Durchführung des erfindungsgemäßen Verfahrens als Ausgangsstoffe zu verwendenden Triazolyl-ketone sind durch die Formel (II) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden.

Die Triazolyl-ketone der Formel (II) sind teilweise bekannt (vgl. z.B. DE-OS 24 31 407 [LeA 15 735], DE-OS 28 20 361 [LeA 18 843], DE-OS 29 51 164 [LeA 20 067], DE-OS 29 06 061 [LeA 19 393] und DE-OS 30 10 560); teilweise sind sie Gegenstand einer eigenen älteren Patentanmeldung, die noch nicht veröffentlicht ist (vgl.die deutsche Patentanmeldung P 31 45 857 vom 19.11.1981 [LeA 21 383]); teilweise sind sie neu. Noch nicht bekannt sind die Triazolylketone der Formel

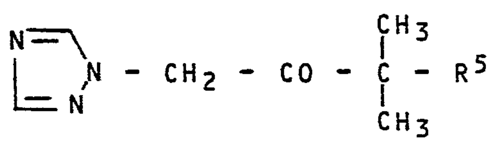 (IV)

in welcher

$R^5$ für Alkenyl mit 2 bis 4 Kohlenstoffatomen, insbesondere Vinyl, steht.

Die neuen Triazolyl-ketone der Formel (IV) werden erhalten, indem man Monochlormethylketone der Formel

$$Cl-CH_2-CO-C(CH_3)_2-R^5 \qquad (V)$$

in welcher

$R^5$ die oben angegebene Bedeutung hat,

in üblicher Weise mit 1,2,4-Triazol in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 150°C umsetzt.

Die Monochlormethylketone der Formel (V) sind ebenfalls nicht bekannt; sie sind jedoch Gegenstand einer eigenen älteren Patentanmeldung, die noch nicht veröffentlicht ist (vgl. die deutsche Patentanmeldung P 30 49 461 vom 30.12.1980 [LeA 20 764]). Die Monochlormethylketone der Formel (V) können danach erhalten werden, indem man 1,1-Dichloralkene der Formel

$$Cl_2C = CH - \underset{CH_3}{\overset{CH_3}{\underset{|}{\overset{|}{C}}}} - R^5 \qquad (VI)$$

in welcher

$R^5$ die oben angegebene Bedeutung hat,

mit Alkaliphenolaten umsetzt und das dabei erhaltene Reaktionsprodukt einer sauren Hydrolyse unterwirft.

Die neuen Triazolyl-ketone der Formel (IV) stellen allgemein interessante Zwischenprodukte dar; in entsprechenden Aufwandmengen zeigen sie auch fungizide Eigenschaften.

Die außerdem für das erfindungsgemäße Verfahren als Ausgangsstoffe zu verwendenden Phenoxyethyl-Derivate sind durch die Formel (III) allgemein definiert. In dieser Formel steht Ar vorzugsweise für die Reste, die bereits im Zusammenhang mit der Beschreibung der erfindungsgemäßen Stoffe der Formel (I) vorzugsweise für diesen Substituenten genannt wurden. Z steht vorzugsweise für eine elektronen-

ziehende Abgangsgruppierung, wie insbesondere Halogen oder p-Methylphenylsulfonyloxy, die Gruppierung $-O-SO_2-OR$ oder $-NR_3$ und andere , wobei R beispielsweise für Alkyl mit 1 bis 4 Kohlenstoffatomen steht.
Die Phenoxyethyl-Derivate der Formel (III) sind allgemein bekannte Verbindungen der organischen Chemie.

Für das erfindungsgemäße Verfahren kommen als Verdünnungsmittel inerte organische Lösungsmittel infrage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wieEssigester; Formamide, wie Dimethylformamid; sowie Dimethylsulfoxid.

Das erfindungsgemäße Verfahren wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielswesie seien Natrium- und Kaliumhydroxid genannt.

Die Reaktionstemperaturen können bei der Durchführung des erfindungsgemäßen Verfahrens in einem größeren Bereich variiert werden. Im allgemeinen arbeitet man zwischen 0 und 100°C, vorzugsweise zwischen 20 und 100°C.

Bei der Durchführung des erfindungsgemäßen Verfahrens arbeitet man vorzugsweise in äquimolaren Mengen. Die Isolierung der Endprodukte der Formel (I) erfolgt in allgemein üblicher Art und Weise.

Das erfindungsgemäße Verfahren kann auch in einem Zweiphasensystem, wie beispielsweise wässrige Natron- oder Kalilauge/Toluol oder Methylenchlorid, gegebenenfalls unter Zusatz von 0,1 bis 1 Mol eines Phasentransferkatalysators, wie beispielsweise Ammonium- oder Phosphoniumverbindungen, beispielsweise seien Benzyldodecyl-dimethyl-ammoniumchlorid und Triethyl-benzyl-ammoniumchlorid genannt, durchgeführt werden.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (I) etwa 1 Mol eines komplexen Hydrids, wie Natriumborhydrid oder Lithiumalanat ein.Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat,so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 0,3 bis 2 Mol Aluminiumisopropylat ein. Zur Isolierung der reduzierten Verbindungen der Formel (I) wird das überschüssige Lösungsmittel

im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I.bis II. sowie IV.bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) könnnen in einfacher Weise nach üblichen Verfahren erhalten werden, so z.B. durch Lösen des Metallsalzes in Alkohol, z.B. Ethanol und Hinzufügen zur Verbindungen der Formel(I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Venturia-Arten, wie z.B. gegen den Erreger des Apfelschorfs (Venturia inaequalis), oder zur Bekämpfung von Puccinia-Arten, wie z.B. gegen den Erreger des Braunrostes am Weizen (Puccinia recondita), eingesetzt werden. Hervorzuheben ist, daß die erfindungsgemäßen Wirkstoffe nicht nur eine protektive Wirkung entfalten, sondern auch kurativ wirksam sind, d.h. auch nach erfolgter Infektion eingesetzt werden können.

In entsprechenden Aufwandmengen zeigen die erfindungsgemäßen Stoffe auch herbizide Wirksamkeit.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkalinaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-,Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Fomulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

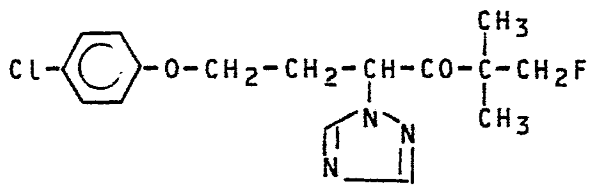

Zu 37,2g (0,2 Mol) 3,3-Dimethyl-4-fluor-1-(1,2,4-triazol-1-yl)-2-butanon in 200 ml Dimethylsulfoxid werden unter Rühren bei 20°C zunächst 11,2g (0,2 Mol) Kaliumhydroxid in 24 ml Wasser und danach 47g (0,2 Mol) 4-Chlorphenoxy-ethylbromid zugegeben. Man läßt über Nacht bei Raumtemperatur nachrühren und gibt das Reaktionsgemisch anschließend auf 400ml Wasser und rührt aus. Der kristalline Niederschlag wird abgesaugt und aus Diisopropylether umkristallisiert. Man erhält 29,3g (43,2 % der Theorie) 6-(4-Chlorphenoxy)-2,2-dimethyl-1-fluor-4-(1,2,4-triazol-1-yl)-3-hexanon vom Schmelzpunkt 46°C.

### Beispiel 2

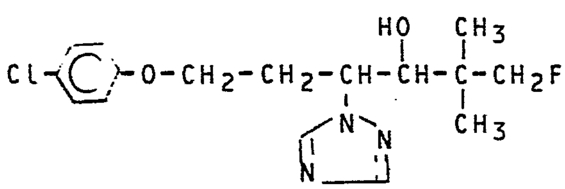

Zu einer Lösung von 20g (0,059Mol) 6-(4-Chlorphenoxy)-2,2-dimethyl-1-fluor-4-(1,2,4-triazol-1-yl)-3-hexanon (Beispiel 1) in 150 ml Methanol werden bei 0 bis 10°C potionsweise 2,8g (0,074 Mol) Natriumborhydrid eingetragen. Man läßt über Nacht bei Raumtemperatur rühren, versetzt mit 120 ml 2n Salzsäure und läßt erneut über Nacht rühren. Anschließend wird das Reaktionsgemisch mit Natriumhydrogencarbonat neutralisiert und mit Methylenchlorid extrahiert. Die organische Phase wird mit Wasser gewaschen, über Natriumsulfat getrocknet und im Vakuum eingeengt. Man erhält 20g (99,5 % der Theorie) 6-(4-Chlorphenoxy)-2,2-dimethyl-1-fluor-4-(1,2,4-triazol-1-yl)-3-hexanol vom Schmelzpunkt 82-88°C.

In entsprechender Weise werden die Verbindungen der folgenden Tabelle der allgemeinen Formel

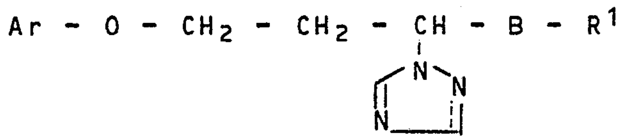

(I)

erhalten:

| Bsp. Nr. | Ar | B | $R^1$ | Schmelzpunkt(°C) $n_D^{20}$ |
|---|---|---|---|---|
| 3 | 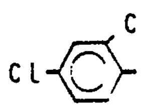 | CO | $-C(CH_3)_2CH_2F$ | 1,5408 |
| 4 | 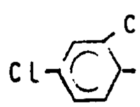 | CH(OH) | $-C(CH_3)_2CH_2F$ | 63-78 |
| 5 | 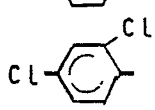 | CH(OH) | $-C(CH_3)_2CH(CH_3)_2$ | 140-42 |
| 6 | 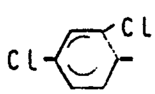 | CH(OH) | $-C(CH_3)_2CH=CH_2$ | 170-8 (xHCl) |
| 7 | 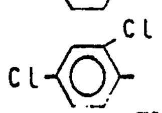 | CO | $-C(CH_3)_2CH(CH_3)_2$ | 60 |
| 8 | 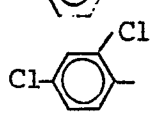 | CH(OH) | 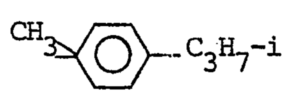 | 120-22 (xHCl) |
| 9 | 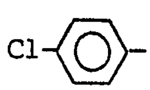 | CH(OH) | $-C(CH_3)_2-CH_2-C(CH_3)_3$ | 79-82 |
| 10 | 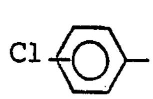 | CH(OH) | $-C(CH_3)_2-CH=CH_2$ | 194-201 (xHCl) |
| 11 | 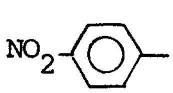 | CH(OH) | $-C(CH_3)_2-CH=CH_2$ | 104-06 |

Verwendungsbeispiele

In den nachfolgenden Beispielen werden die nachstehend angegebenen Verbindungen als Vergleichssubstanzen eingesetzt:

(A) 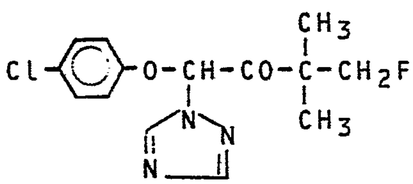

(B) 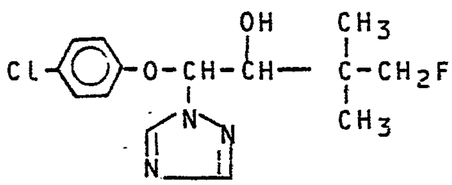

(C) 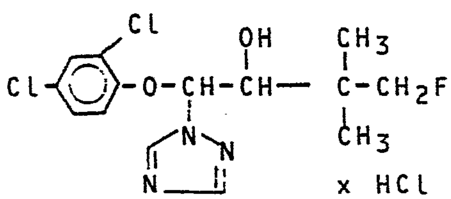

x HCl

(D) 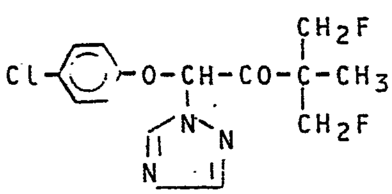

Beispiel A

Venturia-Test (Apfel) / kurativ

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20°C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2 und 4.

Beispiel B

Puccinia-Test (Weizen) / protektiv /

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 1, 2 und 4.

Patentansprüche

1. Phenoxypropyltriazolyl-ketone und -carbinole der allgemeinen Formel

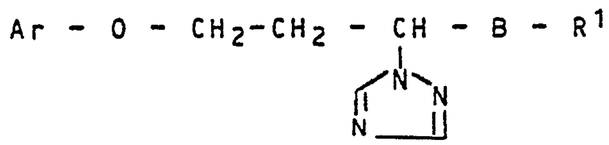

(I)

in welcher

Ar für gegebenenfalls substituiertes Phenyl steht,

B für die Ketogruppe oder eine CH(OH)-Gruppierung steht und

$R^1$ für substituiertes tert.-Butyl, gegebenenfalls substituiertes Phenyl oder gegebenenfalls substituiertes Cycloalkyl steht,

sowie deren Säureadditions-Salze und Metallsalz-Komplexe.

2. Verbindungen der allgemeinen Formel I in Anspruch 1, wobei

Ar für gegebenenfalls einfach bis zweifach, gleich oder verschieden durch Fluor, Chlor, Brom, Methyl, Ethyl, Trifluormethyl, Pentafluorethyl, Methoxy, Ethoxy, Nitro, Phenyl und Chlorphenyl substituiertes Phenyl steht,

B für die Ketogruppe oder eine CH(OH)-Gruppierung steht,

$R^1$ für gegebenenfalls einfach bis zweifach, gleich doer verschieden durch die bei Ar bereits genannten Phenylsubstituenten substituiertes Phenyl steht, sodann für jeweils gegebenenfalls durch Methyl, Ethyl oder Isopropyl substituiertes Cyclopropyl, Cyclopentyl oder Cyclohexyl, sowie für die Gruppierungen

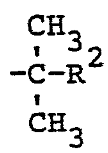 und 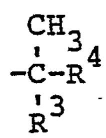 steht, wobei

$R^2$ für Alkyl mit 2 bis 5 Kohlenstoffatomen, Fluormethyl, Chlormethyl oder Vinyl steht und

$R^3$ und $R^4$ für Fluormethyl oder Chlormethyl stehen.

3. Verfahren zur Herstellung von Phenoxypropyltriazolylketonen und -carbinolen der allgemeinen Formel

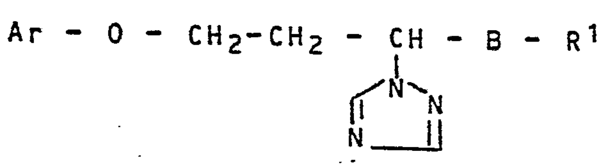

(I)

in welcher

Ar für gegebenenfalls substituiertes Phenyl steht,

B für die Ketogruppe oder eine CH(OH)-Gruppierung steht und

$R^1$ für substituiertes tert.-Butyl, gegebenenfalls substituiertes Phenyl oder gegebenenfalls substituiertes Cycloalkyl steht,

dadurch gekennzeichnet, daß man Triazolyl-ketone der Formel

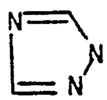 $- CH_2 - CO - R^1$ (II)

in welcher

$R^1$ die oben angegebene Bedeutung hat,

mit Phenoxyethyl-Derivaten der Formel

$$Ar - O - CH_2 - CH_2 - Z \quad (III)$$

in welcher

Ar die oben angegebene Bedeutung hat und

Z für eine elektronenanziehende Abgangsgruppierung steht,

in Gegenwart einer Base und in Gegenwart eines organischen Verdünnungsmittels, oder in einem wässrig-organischen Zweiphasensystem in Gegenwart eines Phasentransferkatalysators, umsetzt und gegebenenfalls noch die so erhaltenen Keto-Derivate der Formel (I) nach bekannten Methoden in

üblicher Weise reduziert, und weiterhin an die so erhaltenen Verbindungen gegebenenfalls anschließend eine Säure oder ein Metallsalz addiert.

4. Schädlingsbekämpfungsmittel, gekennzeichnet durch einen Gehalt an mindestens einem Phenoxypropyltriazolyl-keton oder -carbinol der Formel (I).

5. Verwendung von Phenoxypropyltriazolyl-ketonen oder -carbinolen der Formel (I) zur Bekämpfung von Schädlingen.

6. Verfahren zur Bekämpfung von Schädlingen, dadurch gekennzeichnet, daß man Phenoxypropyltriazolyl-ketone oder -carbinole der Formel (I) auf Schädlinge und/oder ihren Lebensraum einwirken läßt.

7. Verfahren zur Herstellung von Schädlingsbekämpfungsmitteln, dadurch gekennzeichnet, daß man Phenoxypropyltriazolyl-ketone oder -carbinole der Formel (I) mit Streckmitteln und/oder oberflächenaktiven Mitteln vermischt.

8. Triazolyl-ketone der allgemeinen Formel

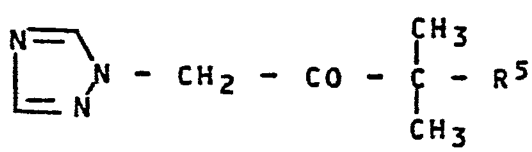

(IV)

in welcher

$R^5$ für Alkenyl mit 2 bis 4 Kohlenstoffatomen steht.

9. Verbindungen gemäß Formel IV in Anspruch 8, wobei $R^5$ für die Vinyl-Gruppe steht.

0109476

Europäisches Patentamt

# EUROPÄISCHER RECHERCHENBERICHT

Nummer der Anmeldung

EP 83 10 2103

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 031 911 (BAYER)<br>* Ansprüche * | 1-4,6,7 | C 07 D 249/08<br>A 01 N 43/64 |
| Y | EP-A-0 032 200 (BAYER)<br>* Ansprüche * | 1-7 | |
| Y | EP-A-0 040 350 (BASF)<br>* Ansprüche * | 1-7 | |
| P,Y | EP-A-0 054 865 (BAYER)<br>* Ansprüche * | 1-7 | |
| D,Y | EP-A-0 055 833 (BAYER)<br>* Ansprüche * | 1-7 | |

RECHERCHIERTE SACHGEBIETE (Int. Cl. 3)

C 07 D 249/00

Der vorliegende Recherchenbericht wurde für alle Patentansprüche erstellt.

| Recherchenort | Abschlußdatum der Recherche | Prüfer |
|---|---|---|
| DEN HAAG | 13-07-1983 | CREMERS K. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument